# EUROPEAN PATENT SPECIFICATION

(11) **EP 1 623 997 B1**
(45) Date of publication and mention of the grant of the patent: **22.04.2015**
(21) Application number: 04728755.2
(22) Date of filing: 22.04.2004
(51) Int. Cl.: C07K 16/46, A61K 39/395

(54) **RECOMBINANT ANTIBODIES AND FRAGMENTS RECOGNISING GANGLIOSIDE N-GLYCOLYL-GM3 AND USE THEREOF IN THE DIAGNOSIS AND TREATMENT OF TUMOURS**
REKOMBINANTE ANTIKÖRPER UND FRAGMENTE, DIE GANGLIOSID-N-GLYCOLYL-GM3 ERKENNEN UND DEREN VERWENDUNG BEI DER DIAGNOSE UND BEHANDLUNG VON TUMOREN
ANTICORPS DE RECOMBINAISON ET FRAGMENTS RECONNAISSANT LE GANGLIOSIDE N-GLYCOLYL GM3 ET LEUR UTILISATION DANS LE DIAGNOSTIC ET LE TRAITEMENT DES TUMEURS

(30) Priority: 23.04.2003 CU 20030092
(43) Date of publication of application: 08.02.2006
(60) Divisional application: 10006891.5
(73) Proprietor: Centro de Inmunologia Molecular, Ciudad Habana 12100 (CU)
(72) Inventor: ROQUE NAVARRO, Loudes, Tatiana, CP 11300 C.Habana (CU); MATEO DE ACOSTA DEL RIO, Cristina, María, Ciudad de la Habana 10800 (CU); RODRIGUEZ GONZALEZ, Mabel, Consejal Veiga No 110, Víbora 10 de Octubre, Ciudad de la Habana (CU); ROJAS DORANTES, Gertrudis, Víbora, 10 de Octubre, CP 10500 C.Habana (CU); TALAVERA PEREZ, Ariel, CP10400 C. Habana (CU); MORENO FRIAS, Ernesto, 5ta Avenida, Edificio BBE, C. Habana, 12100 (CU)
(74) Representative: Jansen, Cornelis Marinus
(86) International application number: PCT/CU2004/000006
(87) International publication number: WO 2004/094477

(56) References cited:
- EP-A2- 1 013 761
- EP-B1- 0 972 782
- DATABASE EPOQUE MEDLINE [Online] 14 July 2004 MARQUINA G. ET AL.: 'Gangliosides expressed in human breast cancer', XP002992864 Database accession no. (NLM8912852) & CANCER RESEARCH vol. 56, no. 22, 1995, pages 5165 - 5172
- MARGALIT H ET AL: "PREDICTION OF IMMUNODOMINANT HELPER T CELL ANTIGENIC SITES FROM THE PRIMARY SEQUENCE", JOURNAL OF IMMUNOLOGY, AMERICAN ASSOCIATION OF IMMUNOLOGISTS, US, vol. 138, no. 7, 1 April 1987 (1987-04-01) , pages 2213-2229, XP002017181, ISSN: 0022-1767
- CHESTER K A ET AL: "CLINICAL APPLICATIONS OF PHAGE-DERIVED SFVS AND SFV FUSION PROTEINS", DISEASE MARKERS, WILEY, CHICHESTER, GB, vol. 16, no. 1-2, 1 January 2000 (2000-01-01), pages 53-62, XP009084098, ISSN: 0278-0240
- DARVEAU R P ET AL: "EXPRESSION OF ANTIBODY FRAGMENTS IN ESCHERICHIA-COLI", JOURNAL OF CLINICAL IMMUNOASSAY, THE SOCIETY, WAYNE, MI, US, vol. 15, no. 1, 21 March 1992 (1992-03-21) , pages 25-29, XP009084099, ISSN: 0736-4393
- KABAT E A ET AL: "Identical V region amino acid sequences and segments of sequences in antibodies of different specificities. Relative contributions of VH and VL genes, minigenes, and complementarity-determining regions to binding of antibody-combining sites.", JOURNAL OF IMMUNOLOGY (BALTIMORE, MD. : 1950) 1 SEP 1991 LNKD- PUBMED:1908882, vol. 147, no. 5, 1 September 1991 (1991-09-01), pages 1709-1719, ISSN: 0022-1767
- DATABASE MEDLINE [Online] US NATIONAL LIBRARY OF MEDICINE (NLM), BETHESDA, MD, US June 1993 WINTER G ET AL: 'Humanized antibodies.' Database accession no. NLM8397764 & IMMUNOLOGY TODAY JUN 1993 LNKD- PUBMED:8397764, vol. 14, no. 6, June 1993 (1993-06), pages 243-246, ISSN: 0167-5699

### SEQUENCE LISTING

<110> CENTRO DE INMUNOLOGIA MOLECULAR CENTRO DE INMUNOLOGIA MOLECULAR
<120> Recombinant antibodies and fragments which recognize N glycolil GM3
   ganglioside and its use in diagnosis and treatment of tumors.
<130> CU 92/2003
<140> CU 92/2003
   <141> 2003-04-23
<160> 58
<170> PatentIn version 3.2
<210> 1
   <211> 5
   <212> PRT
   <213> Mus musculus
<220>
   <221> DOMAIN
   <222> (1)..(5)
<400> 1
   Ser Tyr Trp Ile His
   1               5
<210> 2
   <211> 17
   <212> PRT
   <213> Mus musculus
<220>
   <221> DOMAIN
   <222> (1)..(17)
<400> 2
   Tyr Ile Asp Pro Ala Thr Ala Tyr Thr Glu Ser Asn Gln Lys Phe Lys
   1               5                   10                  15
   Asp
<210> 3
   <211> 16
   <212> PRT
   <213> Mus musculus
<220>
   <221> DOMAIN
   <222> (1)..(16)
<400> 3
   Glu Ser Pro Arg Leu Arg Arg Gly Ile Tyr Tyr Tyr Ala Met Asp Tyr
   1 5 10 15
<210> 4
   <211> 11
   <212> PRT
   <213> Mus musculus
<220>
   <221> DOMAIN
   <222> (1)..(11)
<400> 4
   Arg Ala Ser Gln Ser Ile Ser Asn Asn Leu His
   1 5 10
<210> 5
   <211> 7
   <212> PRT
   <213> Mus musculus
<220>
   <221> DOMAIN
   <222> (1)..(7)
<400> 5
   Tyr Ala Ser Gln Ser Ile Ser
   1 5
<210> 6
   <211> 9
   <212> PRT
   <213> Mus musculus
<220>
   <221> DOMAIN
   <222> (1)..(9)
<400> 6
   Gln Gln Ser Asn Arg Trp Pro Leu Thr
   1 5
<210> 7
   <211> 30
   <212> PRT
   <213> Mus musculus
<220>
   <221> DOMAIN
   <222> (1)..(30)
<400> 7
   Gln Val Gln Leu Gln Gln Ser Gly Asn Glu Leu Ala Lys Pro Gly Ala
   1 5 10 15
   Ser Met Lys Met Ser Cys Arg Ala Ser Gly Tyr Ser Phe Thr
   20 25 30
<210> 8
   <211> 14
   <212> PRT
   <213> Mus musculus
<220>
   <221> DOMAIN
   <222> (1)..(14)
<400> 8
   Trp Leu Lys Gln Arg Pro Asp Gln Gly Leu Glu Trp Ile Gly
   1 5 10
<210> 9
   <211> 32
   <212> PRT
   <213> Mus musculus
<220>
   <221> DOMAIN
   <222> (1)..(32)
<400> 9
   Lys Ala Ile Leu Thr Ala Asp Arg Ser Ser Asn Thr Ala Phe Met Tyr
   1 5 10 15
   Leu Asn Ser Leu Thr Ser Glu Asp Ser Ala Val Tyr Tyr Cys Ala Arg
   20 25 30
<210> 10
   <211> 11
   <212> PRT
   <213> Mus musculus
<220>
   <221> DOMAIN
   <222> (1)..(11)
<400> 10
   Trp Gly Gln Gly Thr Thr Val Thr Val Ser Ser
   1 5 10
<210> 11
   <211> 23
   <212> PRT
   <213> Mus musculus
<220>
   <221> DOMAIN
   <222> (1)..(23)
<400> 11
   Asp Leu Val Leu Thr Gln Ser Pro Ala Thr Leu Ser Val Thr Pro Gly
   1 5 10 15
   Asp Ser Val Ser Phe Ser Cys
   20
<210> 12
   <211> 15
   <212> PRT
   <213> Mus musculus
<220>
   <221> DOMAIN
   <222> (1)..(15)
<400> 12
   Trp Tyr Gln Gln Arg Thr His Glu Ser Pro Arg Leu Leu Ile Lys
   1 5 10 15
<210> 13
   <211> 32
   <212> PRT
   <213> Mus musculus
<220>
   <221> DOMAIN
   <222> (1)..(32)
<400> 13
   Gly Ile Pro Ser Arg Phe Ser Gly Ser Gly Ser Gly Thr Asp Phe Thr
   1 5 10 15
   Leu Ser Ile Ile Ser Val Glu Thr Glu Asp Phe Gly Met Tyr Phe Cys
   20 25 30
<210> 14
   <211> 12
   <212> PRT
   <213> Mus musculus
<220>
   <221> DOMAIN
   <222> (1)..(12)
<400> 14
   Phe Gly Ala Gly Thr Lys Leu Glu Leu Lys Arg Ala
   1 5 10
<210> 15
   <211> 43
   <212> DNA
   <213> Mus musculus
<220>
   <221> prim_transcript
   <222> (1)..(43)
<400> 15
   ggggatatcc accatggaaa ggcactggat ctttttcttc ctg 43
<210> 16
   <211> 30
   <212> DNA
   <213> Mus musculus
<220>
   <221> prim_transcript
   <222> (1)..(30)
<400> 16
   ggggctagct gaggagacgg tgaccgtggt 30
<210> 17
   <211> 48
   <212> DNA
   <213> Mus musculus
<220>
   <221> prim_transcript
   <222> (1)..(48)
<220>
   <221> prim_transcript
   <222> (1)..(43)
<400> 17
   ggggatatcc accatggtat ttcctacacc tcagattacc ttggactt 48
<210> 18
   <211> 35
   <212> DNA
   <213> Mus musculus
<220>
   <221> prim_transcript
   <222> (1)..(35)
<400> 18
   agcgtcgact tacgtttcag ctccagcttg gtccc 35
<210> 19
   <211> 42
   <212> DNA
   <213> Mus musculus
<220>
   <221> prim_transcript
   <222> (1)..(42)
<400> 19
   gtccagcttg tgcagtctgg ggctgaagtg gtaaaacctg gg 42
<210> 20
   <211> 30
   <212> DNA
   <213> Mus musculus
<220>
   <221> prim_transcript
   <222> (1)..(30)
<400> 20
   ggggctagct gaggagacgg tgaccgtggt 30
<210> 21
   <211> 42
   <212> DNA
   <213> Mus musculus
<220>
   <221> prim_transcript
   <222> (1)..(42)
<400> 21
   cccaggtttt accacttcag ccccagactg cacaagctgg ac 42
<210> 22
   <211> 43
   <212> DNA
   <213> Mus musculus
<220>
   <221> prim_transcript
   <222> (1)..(43)
<400> 22
   ggggatatcc accatggaaa ggcactggat ctttctcttc ctg 43
<210> 23
   <211> 27
   <212> DNA
   <213> Mus musculus
<220>
   <221> prim_transcript
   <222> (1)..(27)
<400> 23
   ggggcctcag tgagggtgtc ctgcagg 27
<210> 24
   <211> 30
   <212> DNA
   <213> Mus musculus
<220>
   <221> prim_transcript
   <222> (1)..(30)
<400> 24
   ggggctagct gaggagacgg tgaccgtggt 30
<210> 25
   <211> 27
   <212> DNA
   <213> Mus musculus
<220>
   <221> prim_transcript
   <222> (1)..(27)
<400> 25
   cctgcaggac accctcactg aggcccc 27
<210> 26
   <211> 43
   <212> DNA
   <213> Mus musculus
<220>
   <221> prim_transcript
   <222> (1)..(43)
<400> 26
   ggggatatcc accatggaaa ggcactggat ctttctcttc ctg 43
<210> 27
   <211> 33
   <212> DNA
   <213> Mus musculus
<220>
   <221> prim_transcript
   <222> (1)..(33)
<400> 27
   cactggttaa gacaggcacc tggccagggt ctg 33
<210> 28
   <211> 30
   <212> DNA
   <213> Mus musculus
<220>
   <221> prim_transcript
   <222> (1)..(30)
<400> 28
   ggggctagct gaggagacgg tgaccgtggt 30
<210> 29
   <211> 33
   <212> DNA
   <213> Mus musculus
<220>
   <221> prim_transcript
   <222> (1)..(33)
<400> 29
   cagaccctgg ccaggtgcct gtcttaacca gtg 33
<210> 30
   <211> 43
   <212> DNA
   <213> Mus musculus
<220>
   <221> prim_transcript
   <222> (1)..(43)
<400> 30
   ggggatatcc accatggaaa ggcactggat ctttctcttc ctg 43
<210> 31
   <211> 21
   <212> DNA
   <213> Mus musculus
<220>
   <221> prim_transcript
   <222> (1)..(21)
<400> 31
   ctggaatggg ttggatacat t 21
<210> 32
   <211> 30
   <212> DNA
   <213> Mus musculus
<220>
   <221> prim_transcript
   <222> (1)..(30)
<400> 32
   ggggctagct gaggagacgg tgaccgtggt 30
<210> 33
   <211> 21
   <212> DNA
   <213> Mus musculus
<220>
   <221> prim_transcript
   <222> (1)..(21)
<400> 33
   aatgtatcca acccattcca g 21
<210> 34
   <211> 43
   <212> DNA
   <213> Mus musculus
<220>
   <221> prim_transcript
   <222> (1)..(43)
<400> 34
   ggggatatcc accatggaaa ggcactggat ctttctcttc ctg 43
<210> 35
   <211> 30
   <212> DNA
   <213> Mus musculus
<220>
   <221> prim_transcript
   <222> (1)..(30)
<400> 35
   tatcaacaaa aaccaggtca gtctccaagg 30
<210> 36
   <211> 35
   <212> DNA
   <213> Mus musculus
<220>
   <221> prim_transcript
   <222> (1)..(35)
<400> 36
   agcgtcgact tacgtttcag ctccagcttg gtccc 35
<210> 37
   <211> 30
   <212> DNA
   <213> Mus musculus
<220>
   <221> prim_transcript
   <222> (1)..(30)
<400> 37
   ccttggagac tgacctggtt tttgttgata 30
<210> 38
   <211> 48
   <212> DNA
   <213> Mus musculus
<220>
   <221> 1prim_transcript
   <222> (1)..(48)
<400> 38
   ggggatatcc accatggtat ttcctacacc tcagattacc ttggactt 48
<210> 39
   <211> 21
   <212> DNA
   <213> Mus musculus
<220>
   <221> prim_transcript
   <222> (1)..(21)
<400> 39
   atttctgggg tcccctccag g 21
<210> 40
   <211> 35
   <212> DNA
   <213> Mus musculus
<220>
   <221> prim_transcript
   <222> (1)..(35)
<400> 40
   agcgtcgact tacgtttcag ctccagcttg gtccc 35
<210> 41

## Description

### Technical Field.

The present invention relates to the field of immunology and in particular with the obtaining less immunogenic immunoglobulins using genetic engineering technologic to use in the cancer treatment. The present invention is related specifically with the peptide sequences which codified a monoclonal antibody or fragments derived which recognize antigens containing the N-glycolil GM3 ganglioside and no other related gangliosides as N-glycolil or N-acetyl, and nor sulphates glycolipids

### Background of the invention.

The gangliosides are glycosphingolipids which contain sialic acid and they are present in plasmatic membranes of vertebrates (Stults CLM et. al, Methods Enzymology 179:167-214, 1989). Some of these molecules has been reported in the literature as tumour associated antigens or tumoral markers (Hakamori SH: Curr Opin Immunol 3:646-653, 1991), then It has been described the use of anti gangliosides antibodies in the diagnosis and therapeutic of cancer. (Hougton AN et. al, NAS USA 82:1242-1246, 1985; Zhang S et al. Int J Cancer 73:42-49, 1997). The more commonly sialic acids in mammalian cells are N-acetyl (NAc) and the N-glycolil (NGc) (Corfield AP et. al, Cell Biol Monogr 10:5-50, 1982). Generally the NGc is not expressed in human and chicken normal tissues but it is widely distributed in other vertebrates (Leeden RW et. al, Biological Role of Sialic Acid. Rosemberg A and Shengtrund CL (Eds). Plenum Press, New York, 1-48, 1976, Kawai T et. al, Cancer Research 51:1242-1246, 2001). However reports from the literature show that the anti-NGc antibodies recognize some human tumors and tumoral cell lines (Higashi H et. al, Jpn J. Cancer Res. 79:952-956, 1988, Fukui Y et. al, Biochem Biophys Res Commun 160:1149-1154, 1989). An increase in the GM3 (NGc) ganglioside level has been found in human breast cancer (Marquina G et. al, Cancer Research 56:5165-5171, 1996), which makes very attractive the use of this molecule as target for cancer therapy. The filed patent EP 0972782 A1 describes the murine monoclonal antibody produced by the hybridoma deposited according to the Budapest treaty under the access number ECACC 98101901. This monoclonal antibody has an IgG1 isotype and was generated by the immunization of Balb/c mice with the NGcGM3 ganglioside hydrophobically conjugated with low density lipoproteins (VLDL) and in presence of Freund adjuvant, said antibody binds preferentially to the NGcGM3 ganglioside and recognizes an antigen expressed in human breast and melanoma tumors. (Carr A et. al, Hybridoma 19:3:241-247, 2000). This antibody showed a strong cytolitic activity on cells bearing said sequence a property that could make it a therapeutic tool.

Moreover it is known that the neovascularization induced by the tumors constitute one of the main parameters in the control of tumoral growth. This has direct research towards the search for new therapeutic weapons related with the inhibition of the antigenic process. There are experimental evidences of the effect of the 14F7 antibody concerning this process. Said evidences have been found in matrix gel assay (Angiogenesis. Vol. 4, Pags. 113-121, 2001; Anti-Cancer Res. Vol.18, Pags.783-790, 1998). Matrix gel is a mixture of proteins of the basal membrane and the extra cellular matrix that surrounds the endothelial cells. Its usefulness in the neovascularization process lies in the physiological importance of the endothelium-matrix interactions during the development of the new blood vessels. Likewise components of this gel such as laminine are considered important markers of this process.

This model type has also been used in the study of the mechanisms related with tumor induced angiogenesis allowing to evaluate the pharmaceuticals capable of modulating the events directly related with the tumor thus influencing its growth and metastasis.

The use on non-human monoclonal antibodies such as the murine 14F7 has certain disadvantages for human treatment particularly in repetitive therapeutic schemes. For example the murine monoclonal antibodies have a relatively short blood half life. Additionally they lose important immunological properties such as the effector functions when used in humans.

And what could be of greater importance, the murine monoclonal antibodies contain considerable amino acid sequences that are immunogenic when injected in humans. Numerous studies have shown that after the administration of a foreign antibody the immune response produced in the patient can be considerably strong and substantially eliminate the therapeutic effect of the antibody after an initial treatment. Moreover, even after administering treatment to a patient with a murine monoclonal antibody later treatments with non related murine antibodies could be ineffective and even dangerous due to the cross reactivity known as HAMA response (Khazaeli, M.B., et. al, Journal of Immunotherapy 15: 42-52, 1994).

Mateo et. al (US Patent No. US 5 712 120) describe a procedure for the reduction of immunogenicity of the murine antibodies. The antibody modified by the method described by Mateo et. al (Mateo C et. al, Hybridoma 19:6:463-471, 2000), retains the recognition and binding capacity to the antigen of the original antibody and results less immunogenic, which increases its therapeutic usefulness. Be this procedure one obtains, with a small number of mutations, modified antibodies that show a reduction of the immunogenicity when compared with the chimeric antibody.

From the above it is inferred the of obtaining versions of the therapeutic antibodies that are less immunogenic, obtained in a simple economic way and that result adequate for the manufacturing of therapeutic formulations for human use.

It is also known that the use of antibody fragments has been very useful in the immunodiagnosis of diseases. Ira Pastan among others (European patent filed EP 0796334 A1) describes the construction of Fv type single chain fragments using the variable regions of antibodies that speficifically recognize the carbohydrate related with the Lewis Y antigen. Based on said fragments the author develops a method for detecting cells bearing said antigen and also provides evidences of the inhibiting effect that these fragments have on the cells bearing the antigen.

The knowledge of the binding site of the 14F7 monoclonal antibody results interesting form the practical and theoretical point of view due to the potential of this antigen in the immunotherapy of diverse diseases. Souriau, C et. al in Expert Opin. Biol. Ther. 1, 845-855, 2001 and Chester, K. A., et. al in Dis. Markers. 16, 53-62, 2000, show that the use of antibody fragments of the scFv type could result of great therapeutic use taking advantage of its pharmacological properties such as a better penetration in the tumoral tissue.

The construction of mini-libraries of antibody fragments exposed on philamentous phages from hybridomas allows the selection of molecules with the capacity of specific recognition of the antigen within this repertoire. The objective is obtaining rapidly antibody fragments that combine the recognition of the original antibody with a smaller molecular size and the capacity of being produced by bacteria host. This technique allows to outstand the multiple variants of antibody fragments that are non functional or impossible to be produced in bacterias that are obtained during the isolation and manipulation of the genes of the variable regions of a hybridoma by conventional clonation techniques (Roovers, R. C. et. al, Br. J. Cancer. 78, 1407-1416, 1998).

The technology of antibody fragment presentation in filamentous phages offers a unique opportunity to access the biding site of the antibody as well as for introducing modifications in the antibody with the objective of improving its affinity (Chames, P. et. al, J. Immunol. 161, 5421-5429, 1998, Lamminmaki, U et. al, J. Mol. Biol. 291, 589-602, 1999, Parhami-Seren et. al, J. Immunol. Methods. 259, 43-53, 2002) or to modulate its specificity (Iba, Y. et. al, Prot. Engn., 11, 361-370,1998, Miyazaki, C. et. al, Prot. Engn. 12, 407-415,1999, Darveau, R. P. et. al, J. Clin. Immunoassay. 15, 25-29, 1992).

The expression of antibody fragments in filamentous phages allows the exchange of chains (Lantto, J. et. al, Methods Mol. Biol. 178, 303-316, 2002), that is the combination of different VLs, with the original antibody VH or vice versa thus allowing to study the role that each one of the chains plays in the recognition of the antigen and its influence in the affinity of the antibody (Kabat, E. A., Wu, T. T., J. Immunol. 147, 1709-1719, 1991, Barbas III, C. F., Lerner, R. A. Methods: A companion to Methods in Enzymology 2, 119-124,1991). The exchange of chains allows modifying the properties of the binding site and also combining immunoglobulin sequences from different species and selecting those variants that conserve the recognition specificity (Klimka, A. et. al, Br. J. Cancer. 83, 252-260, 2000, Steinberger, P. et. al, J. Biol. Chem. 275,36073-36078, 2000, Rader, C. et. al, Proc. Natl. Acad. Sci. USA. 95,8910-8915, 1998, Beiboer, S. W. H. et. al, J. Mol. Biol. 296,833-849, 2000).

EP0972782 A1 relates to the branch of immunology and human medicine, in particular to the generation and selection of a monoclonal antibody which recognizes the oligossaccharide N-glycolylated-glucose sialic acid (N-GcNeu-Gal-Glu) present in malignant tumors.

The present invention refers to a modified antibody derived from the murine 14F7 monoclonal antibody which recognizes the original antigen with the same affinity and results less immunogenic when administered to patients. Another finding of the present invention has been obtaining fragments derived from said antibody that comprises variable regions of light chains different from the original but retaining its properties in relation to specificity, affinity and recognition and can be expressed by a bacteria host as soluble molecules as well as presented on the surface of filamentous phages. The fragments derived from the 14F7 antibody are useful as therapeutic weapons. Additionally the expression of scFv type fragments of the 14F7 antibody on the surface of a M13 filamentous phage allows manipulating the binding site and obtaining variants with higher affinity with therapeutic purposes.

One of the objectives of the present invention is the characterization of the antibody fragments that only conserve partially the sequence of the 14F7 but retain its specificity, affinity and recognition properties.

Both the modified antibody and the fragments obtained recognize specifically the tumor cells that express the NGcGM3 antigen thus allowing them to be used in the diagnosis or therapy of said tumors and have the advantage of resulting less immunogenic than the murine antibody from which they were originated.

New therapeutic compositions that comprise the modified 14F7 antibody or its Fv single chain fragments where said antibody or fragments may be bound to a radioactive isotope useful in localizing or treating tumors expressing the antigen are objects of the present invention.

As disclosed herein, a method for the radioimmunodiagnosis or radio immunotherapy of tumors expressing the NGcGM3 ganglioside using the pharmaceutical compositions that comprise the modified 14F7 antibody or its fragments bound to a radioactive isotope.

### Detailed description of the invention.

In the present invention is described the chimeric antibody derived from the murine 14F7 monoclonal antibody produced by the hybridoma with deposit number ECACC 98101901, characterized by the following sequences of the hyper variables regions (CDRs) of the heavy and light chains:
HEAVY CHAIN
   CDR1: SYWIH
   CDR2: YIDPATAYTESNQKFKD
   CDR3: ESPRLRRGIYYYAMDY
LIGHT CHAIN
   CDR1: RASQSISNNLH
   CDR2: YASQSIS
   CDR3: QQSNRWPLT

Preferentially said chimeric antibody is characterized by the following sequences of the framework regions (FRs) of the heavy and light chains:
HEAVY CHAIN
   FR1: QVQLQQSGNELAKPGASMKMSCRASGYSFT
   FR2: WLKQRPDQGLEWIG
   FR3: KAILTADRSSNTAFMYLNSLTSEDSAVYYCAR
   FR4: WGQGTTVTVSS
LIGHT CHAIN
   FR1: DLVLTQSPATLSVTPGDSVSFSC
   FR2: WYQQRTHESPRLLIK
   FR3: GIPSRFSGSGSGTDFTLSIISVETEDFGMYFC
   FR4: FGAGTKLELKRA

Moreover chimeric antibody is characterized by containing the constant region of the IgG1 human heavy chain and the constant region of the Ck human light chain.

Preferably, said modified antibody is characterized by containing the constant region of the IgG1 human heavy chain and the constant region of the Ck human light chain.

Another aspect of the present invention refers to the Fv single chain type fragments derived from the 14F7 antibody that comprise the sequence of the variable region of the heavy chain obtained from the hybridoma (deposit ECACC 98101901) and that conserves the recognition specificity of the 14F7

Preferably said Fv fragments include light chain variable regions derived from non immunized mice or human, different from the light chain variable region produced by the referred hybridoma that allow its production by a bacteria host as antibody fragments presented on filamentous phages or soluble molecules.

The present invention is also related with the cell lines that express the chimeric and humanized antibodies and the bacteria clones that produce the fragments derived from the 14F7.

Moreover the present invention is related with the pharmaceutical compositions for the treatment and/or localization and identification of the malignant breast and melanoma tumors their metastases and recurrences characterized by containing one of the recombinant monoclonal antibodies or its fragments of the present invention as well as an exipient appropriate for its application. Said antibody or fragment could be bound to a radioactive isotope useful in the localization and/or therapy of the malignant tumors. Finally the present invention is related with the use of the described recombinant antibodies for their in manufacturing a pharmaceutical composition useful in the treatment and/or localization and identification of malignant tumors.

The detailed description of how to execute and use the present invention is referred below:

### 1. ADNc synthesis and amplification by PCR (Polymerase Chain Reaction) of the variable regions of the 14F7 murine antibody.

The murine 14F7 antibody was obtained by immunizing Balb/c mice with the GM3 (NeuGc) ganglioside hydrophobically conjugated with low density lipoproteins and in presence of Freund adjuvant (EP 0972782 A1, Carr A et. al, Hybridoma 19:3:241-247, 2000). The RNA is obtained from 10⁶ cells of the hybridoma producing the 14F7 monoclonal antibody (murine IgG1 Mab) that recognizes the NGcGM3 ganglioside using the TRIZOL extraction method (GIBCO BRL, NY), according to the manufacturer's instructions.

The synthesis of the complementary DNA (DNAc) and the amplification of the VH and VL variable regions were performed using the set of reagents: Access RT-PCR (Promega, USA) according to the manufacturer's recommendations. Briefly, the reaction is performed from 5 µg of RNA in presence of 25 pmoles of the dT olygonucleotide designed for hybridizing in the poly A tail the RNA and the corresponding olygonucleotides the extremes of each variable region: VH and VL. Incubate 10 minutes at 60ºC and later add the mixture containing 0.2 mM of each deoxinucleotide (dNTPs), 1 mM MgSO₄, 5 µ of AMV-Reverse Transcriptase and 5 µ of DNA Polymerase in reaction buffer for a total volume of 50 µL. The samples are incubated for 45 minutes at 48ºC, 2 minutes at 94ºC and later submitted to 40 cycles of PCR with temperatures of 94ºC (30 seconds), 60ºC (1 minute), 68ºC (2 minutes), and with a final incubation of 7 minutes at 68ºC.

### 2. Contruction of chimeric genes and sequencing of the amplified DNAc.

The products of the PCR of the VH and VK are digested with the restriction enzymes *Eco* RV- *Nhe* I, for VH, and *Eco* RV-*Sal* I for VK, and cloned in the respective expression vectors (Coloma MJ et. al, J Immunol Methods 152:89-104, 1992). The VH region is cloned in the PAH4604 vector that has included a IgG1 human constant region and as selection marker the gen resistant to L-histidinol. The VK region is cloned in the vector PAG4622, which bears a gene of resistance to micophenolic acid and a constant human kappa region. The resulting genetic constructions were named 14F7VH-PAH4604 and 14F7VK-PAG4622, respectively.

Both constructions are sequenced by the dideoxinucleotide method (Sanger F et. al, PNAS USA 74:5463-5470, 1979) using the sequence reagent kit T7 DNA Polymerase (Amershan-Pharmacia) according to the protocol described by the manufacturer.

### 3. Expression of the chimeric antibody.

NSO cells are electroporated with 10 µg of 14F7VH-PAH4604 and 10 µg of 14F7VK-PAG4622 linealized with the enzyme *Pvu* I. The DNAs are precipitated with ethanol, mixed and dissolved in 50 µL of PBS (Phospate buffer saline solution). Approximately 10⁷ cells are grown to semi confluence and are collected by centrifugation. The cells are later resuspended in 0.5 mL of PBS together with the DNA in an electroporation tray. After 10 minutes in ice the cells are submitted to a pulse of 200 V and 960 F, and are kept in ice for 10 minutes. The cells are cultured in 96 wells plates in Modified Dulbecco (DMEM-F12) selective culture media, with 10% fetal calf serum (FCS) and 10 mM of L-hystidinol. The transfected clones are made visible past 10 days of adding the selection media.

The production of chimeric immunoglobulin is determined by ELISA from the clones supernatant. For this the polystyrene plates (High binding, Costar) are coated with goat anti human IgG serum in carbonate bicarbonate buffer 100 mM, pH 9.8, all night at 4ºC. The plates are then washed with PBS-Tween (Phosphate buffer saline solution, 0.5% Tween-20, pH 7.5), and the culture supernatant samples diluted in PBS-Tween-FCS are added and incubated during one hour at 37ºC. The plates are washed again in PBS-Tween and are then incubated in goat anti human kappa chain serum conjugated with peroxidase (Jackson), for one hour at 37ºC. Later the plates are washed in the same manner and incubated with a citrate-phosphate buffer solution pH 4.2 that contains the substrate: o-fenilendiamine. After 15 minutes the absortion is measured at 492 nm.

The capacity of the chimeric antibody recognizing the antigen is verified by a competitive ELISA using the NGcGM3 and NacGM3 gangliosides. Briefly, the polyestirene plates (Polysorp, Nunc) are coated with 50 µL of a solution at 4 µg/mL of NGcGM3 or NAcGM3 in methanol and are incubated for one hour at 37ºC. The plates are then blocked with 200 µL of bovine seroalbumin solution (BSA) 1% in Tris-HCl buffer, pH 7.8-8.0 during one hour at 37ºC. After 3 washes with PBS, the samples diluted in Tris-HCl-BSA 1% are added in a concentration range between 2 µg/mL - 0.01 µg/mL in presence of the the biotinilated murine 14F7 antibody 1 mg/mL, at 37ºC during 2h. After the plates are washed with PBS and the reaction is developed with a streptavidine-peroxidase conjugate (Jackson), at 37ºC, 1 h. The absorbance is measured at 492 nm.

### 4. Construction of the humanized Ab 14F7hT by humanization of T epitopes. Prediction of T epitopes.

The sequences of the variable domains of the 14F7 are analyzed with the AMPHI Program (Margalit H et. al, J Immunol 138: 2213-2229, 1987), which allows the identification of the segments of 7 or 11 amino acids with an anphypatic helix structure, which has been related with the T cell immunogenicity. These algorithms predict in this case the fragments related with the presentation of T epitopes in the variable regions of the heavy and light chains of the murine 14F7 monoclonal antibody.

### - Analysis of homology with human immunoglobulins.

The amino acid sequences of the variable domains of the 14F7 are compared with the sequences of variable regions of reported human immunoglobulins to identify the human immunoglobulin that has the highest homology with the murine molecule being analyzed. For this one can use the SWISSPROT data base available in Internet, through the BLAST program (Altschul S F et. al, Nucleic Acids Res 25:3389-3402, 1997).

### - Analysis for the reduction of immunogenicity.

The essence of the method lies in achieving the reduction of the immunogenicity by the rupture or humanization of the possible T epitopes, with a minimum of mutations in the FRs, specifically in those segments that have a amphipatic helix structure excluding those positions involved in the three-dimensional structure of the antigen recognition site. According to the method the sequences of the variable regions VH and VL of the murine immunoglobulin are compared with the human one of highest homology and the residues that differ between the murine and the human sequence are identified only in the amphipatic regions that are within the region of the FRs (Kabat E, Sequences of proteins of immunological interest, Fifth Edition, National Institute of Health, 1991). These "murine" residues will be the ones susceptible of being muted by those that are found in the same position of the human sequence.

The residues present in the FRs positions responsible of the canonic structure, those present in the Vernier zone or the ones participating in the interaction of the VH-VL interphase can not be mutated because it could affect the three-dimensional structure of the variable domain of the antibody and thus affect the binding to the antigen. Additional information about the influence of the substitutions proposed on the tertiary structure can be obtained by molecular modeling of the variable regions.

Since in the analysis of the mutations elements such as the presence of proline residues in the amphipatic helix or the fact that a certain murine residue does not appear in the same position in the most homologous human sequence but is frequent in other human immunoglobulins can be considered it is possible to obtain a version that contains a maximum of mutations, that is, where all the murine residues that differ from the human sequence are mutated but other versions can be obtained with different combinations of mutations.

After identifying on the murine sequence of the 14F7 the possible T epitopes and identifying within these segments the residues different from the human ones, the substitutions are made by the conventional techniques of directed mutagenesis.

### - Cloning and expression of the humanized 14F7hT antibody in the NSO cells.

Once the genetic constructions corresponding to the VH and VL regions of the 14F7hT antibody are obtained by the method described before, they are cloned in the respective expression vectors similarly as describe above in the case of the chimeric antibody construction, obtaining the following genetic constructions: 14F7hTVK-PAG4622 and 14F7hTVH-PAH4604. The transfections of these genes to the NSO cells is exactly under the same conditions described for the chimeric antibody. The clones producing the humanized antibody are also detected by ELISA.

The capacity of specific recognition of the antigen by the humanized antibody is verified by a competitive ELISA using the NGcGM3 and NAcGM3 gangliosides. The procedure is identical to the one described for the chimeric antibody

### 5. Construction of antibody fragments libraries on filamentous phages from the 14F7 hybridoma.

The messenger RNA is isolated from the cells of the 14F7 hybridoma, the complementary DNA is synthesized and the sequences corresponding to the variable regions of the heavy and light chains are separately amplified using two sets of oligonucleotides designed to hybridize with a wide spectrum of murine variable regions. The variable regions of each amplified heavy chain is purified, digested with the appropriate enzymes and linked to the phagemide pHG-1m vector (designed for the presentation of single chain antibodies on the surface of filamentous phages), previously digested with the same enzyme. The products of the binding reaction are purified and introduced by electroporation in bacterias of the TG1 *E. coli* strain. The transformed bacterias constitute a semi-library of heavy chain variable regions of limited diversity (all to them derived from the 14F7 hybridoma). The DNA corresponding to this semi-library is purified and separately linked to collections of variable regions of the light chain (previously purified and digested with the appropriate enzymes). Finally they genetic constructions are introduced in bacterias of the TG1 strain by electroporation, to conform different types of independent libraries. One of them is a mini-library of limited diversity constructed with the variable regions of the light chain, also obtained from the cells of the 14F7 hybridoma. Libraries of chain exchange are also obtained in which the variable regions of the heavy chains from the hybridoma are combined with diverse collections of variable regions of light chains obtained from murine and human lymphocytes.

### 6. Isolation and characterization of the clones producing antibody fragments against N-glycolil GM3.

Colonies of each library are taken at random to produce phages from them using the auxilliar phage M13 K07. The recognition of N-glycolil GM3 for the phages bearing the antibody fragments is analyzed directly by an solid phase immunoenzymatic test (ELISA). Thus the clones producing the functional fragments are localized. Phages bearing antibody fragments from the total mixture of transformed bacteria that forms each library are produced to obtain a preparation rich in functional antibody fragments and explore the diversity of the libraries. The phage mixture obtained is place in contact with the antigen N-glicolil GM3 bound to a solid surface, the phages bearing the antibody fragments with binding capacity are retained and the rest is eliminated by exhaustive washings. The bound phages are eluted by a pH change and are multiplied to obtain a new phage mixture that serves as starting material for a new selection cycle on the antigen. After various selection cycles the recognition of the antibody fragments presented on phages from colonies is analyzed. Thus the additional clones producing functional fragments diluted in the initial library are identified.

The characterization of the antibody fragments produced by the clones includes the analysis of the specificity by ELISA against a panel of related gangliosides, the study of the tumor recognition by immunohistochemistry, the evaluation of its capacity to be produced as soluble functional antibody fragments outside the context of their presentation in phages and the complete sequencing of their variable regions.

### Examples:

In the following examples all the restriction enzymes or modification used as well as the reagents and materials were obtained from commercial source unless otherwise specified.

### Example 1. Obtaining the 14F7 Chimeric Monoclonal Antibody.

The synthesis of the DNAc and the amplification by PCR of the VH and VK murine variable regions was performed with the *Vent* Polymerase enzyme using specific oligonucleotides with restriction sites *Eco* RV-*Nhe* I for VH, and *Eco* RV-*Sal* I for VK. The switch oligonucleotides were the following:
For VH:
   Oligonucleotide 1 (hybrid in the signal peptide): 5' ggg gatatc cacc atg gaa agg cac tgg atc ttt ctc ttc ctg 3'
   Olygonucleotide 2 (hybrid in JH1): 5' ggg gctagc tga gga gac ggt gac cgt ggt 3'
For VK:
   Olygonucleotide 1 (hybrid in the signal peptide): 5' ggg gatatc cacc atg gt(at) t(tc)c (ta)ca cct cag (at)t(ac) ctt gga ctt 3'
   Olygonucleotide 2 (hybrid in VLJ5): 5' agc gtcgac tta cgt ttc agc tcc agc ttg gtc cc 3'

The products of the PCRs of VH and VK were digested with teh restriction enzymes *Eco* RV-*Nhe* I, for VH, and *Eco* RV-*Sal* I for VK, and cloned in their respective expression vectors, PAH4604 and PAG4622, for VH and VK respectively. These vectors are used for the expression of immunoglobulins in mammalian cells and were donated by Sherie Morrison (UCLA, California, USA). The vector PAH4604 has the human IgG1 constant region included and the PAG4622, a human constant kappa region (Coloma J et. al, J Immunol Methods 152:89-104, 1992). Once the VH and VK regions of the 14F7 have been cloned in the previous vectors, the constructions 14F7VH-PAH4604 and 14F7VK-PAG4622 are formed.

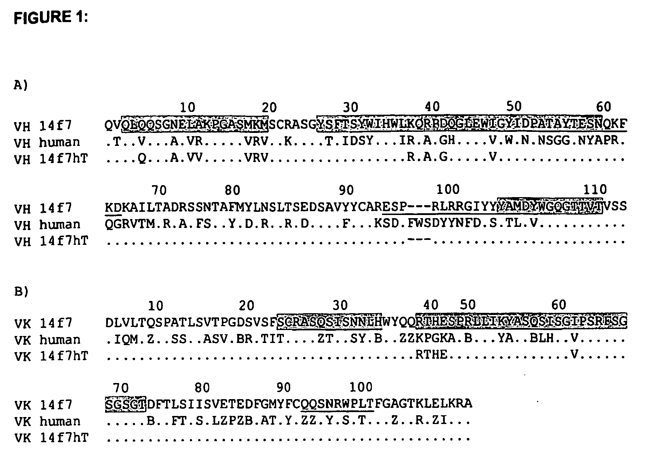

Twelve independent clones were sequenced by the dideoxinucleotide method (Sanger F et al, DNA sequencing with chain-terminating inhibitors. PNAS USA 1979;74:5463-5470) using the T7 DNA Polymerase sequencing kit (Amershan-Pharmacia) according to the protocol described by the manufacturer. The VH and VK sequences have a high relation with the IIB and V subgroups, respectively, (Figures 1A and B) according to the Kabat classification (Kabat E, Sequences of proteins of inmunological interest, Fifth Edition, National Institute of Health, 1991).

NS0 cells were electroporated with 10 µg of 14F7VH-PAH4604 and 10 µg of 14F7VK-PAG4622 linealized with the enzyme *Pvu* I. The DNAs were precipitated with ethanol, mixed and dissolved in 50 µL of PBS. Approximately 10⁷ cells grown until semi confluence were collected by centrifugation and resuspended in 0.5 mL of PBS together with the DNA in the electroporation tray. After 10 minutes in ice the cells were submitted to a pulse of 200 V and 960 F and incubated in ice for 10 minutes. The cells were cultured in 96 wells plates in Dulbecco Modified (DMEM-F12) selected media with 10% FCS and 10 mM of L-hystidinol. The transfected clones are visualized 10 days after adding the selection media. The production of chimeric immunoglobulin is determined by ELISA using the clones supernatants. For this the polyestirene plates (High binding, Costar) are coated with goat anti human IgG serum (Sigma) in carbonate bicarbonate buffer 100 mM, pH 9.8, all night at 4ºC. The plates are then washed with PBS-Tween (Phosphate buffer saline solution, 0.5% Tween-20, pH 7.5), and the culture supernatant samples diluted in PBS-Tween-FCS are added and incubated during one hour at 37ºC. The plates are washed again in PBS-Tween and are then incubated in goat anti human kappa chain serum conjugated with peroxidase (Jackson), for one hour at 37ºC. Later the plates are washed in the same manner and incubated with a citrate-phosphate buffer solution pH 4.2 that contains the substrate: o-fenilendiamine. After 15 minutes the absorption is measured at 492 nm.

### Example 2. Reactivity of the 14F7Q antibody against the NGcGM3 and NAcGM3 antibodies.

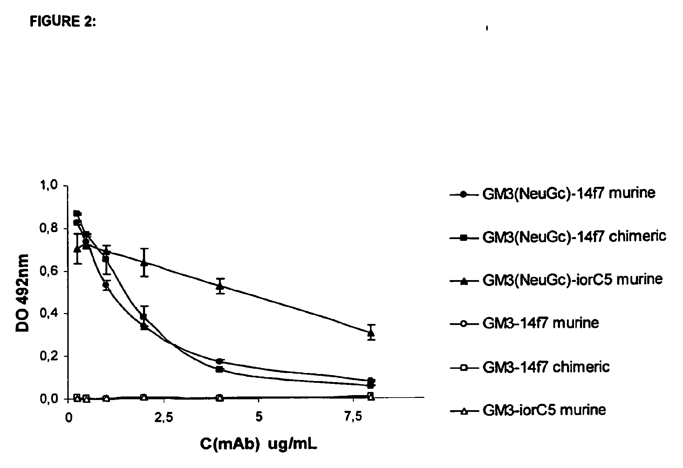

The reactivity of the 14F7 chimeric antibody (Ab) was determined by a competitive ELISA. Figure 2 shows the specificity of the chimeric 14F7 compared with the murine 14F7 in the recognition of polyesterine plates Polysorp, Nunc) coated with the GM3 N-glycolilated ganglioside. Both Abs show at similar concentration a 50 % inhibition of the recognition of the antigen by the murine biotinilated 14F7 Ab. While no immunoreactivity was observed when coating the plates with the N-acetylated variant of the GM3 for either of the antibodies. The murine C5 Mab was used as the irrelevant Ab.

### Example 3. Obtaining different versions of the humanized antibody.

The 14F7 VH and VK sequences were compared with the human sequences data base obtaining the most homologous human sequence of the 14F7 antibody for the VH and VK regions (Fig 1A and B). Additionally in both sequences the amphipatic regions or potential T epitopes were determined. Later the mutations needed to convert the murine VH and VK sequences into human sequences were determined following the this method of humanization of T epitopes. We refer below the maximum possible mutations that can be performed.

In the case of the VH region mutations in the positions 5, 9, 11, 12, 18, 19, 20, 38, 40, 42 and 48 were introduced, where the amino acids Q, N, L, A, M, K, M, K, R, D and I were substituted for V, A, V, V, V, R, V, R, A, G and V, respectively. These mutations were performed by overlapping the PCR products (Kamman M et. al, Nucleic Acids Research 17:5404-5410, 1989), using the oligonucleotides 1 and 2, and the 3 and 4, in the first PCRs, and the results were overlapped in another PCR using oly the olygonucleotides 2 and 4. The sequences of the olygonucleotides used are shown below:
Olygonucleotides for the mutations 5, 9, 11 and 12, of the heavy chain:
Olygonucleotide 1: 5' gtc cag ctt gtg cag tct ggg gct gaa gtg gta aaa cct ggg 3'
Olygonucleotide 2: 5' ggg gctagc tga gga gac ggt gac cgt ggt 3'
Olygonucleotide 3: 5' ccc agg ttt tac cac ttc agc ccc aga ctg cac aag ctg gac 3'
Olygonucleotide 4: 5' ggg gatatc cacc atg gaa agg cac tgg atc ttt ctc ttc ctg 3'

Once the previous mutations are sequenced and verified, mutations in the 18, 19 and 20 positions were introduced in the DNAs that carried them, substituting the amino acids M, K, M for V, R, V, respectively.

Below are shown the olygonucleotides 1 and 2, and los 3 and 4, described for said mutations. The overlapping of the PCRs products was performed as described before. Olygonucleotide for the mutations 18, 19 and 20 of the heavy chain:
Olygonucleotide 1: 5' ggg gcc tca gtg agg gtg tcc tgc agg 3'
Olygonucleotide 2: 5' ggg gctagc tga gga gac ggt gac cgt ggt 3'
Olygonucleotide 3: 5' cct gca gga cac cct cac tga ggc ccc 3'
Olygonucleotide 4: 5' ggg gatatc cacc atg gaa agg cac tgg atc ttt ctc ttc ctg 3'

Likewise once the previous mutations are sequenced and verified, mutations R, A, G were introduced in the positions 38, 40 and 42 in the DNAs that carried them, substituting the amino acids K, R, D, respectively.

Below are shown the olygonucleotides 1 and 2, and 3 and 4, described for these mutations. The overlapping of the PCRs products was performed as described before. Olygonucleotides for the mutations 38, 40 and 42 of the heavy chain:
Olygonucleotide 1: 5' cac tgg tta aga cag gca cct ggc cag ggt ctg 3'
Olygonucleotide 2: 5' ggg gctagc tga gga gac ggt gac cgt ggt 3'
Olygonucleotide 3: 5' cag acc ctg gcc agg tgc ctg tct taa cca gtg 3'
Olygonucleotide 4: 5' ggg gatatc cacc atg gaa agg cac tgg atc ttt ctc ttc ctg 3'

Finally after verifying by sequence the prior mutations, a mutation in position 48 was introduced in the DNAs bearing them substituting the amino acid I for V. Below are shown the olygonucleotides 1 and 2, and 3 and 4, used in this mutation. The overlapping of the PCRs products was performed as described before.

Olygonucleotides for the mutation 48 of the heavy chain:
Olygonucleotide 1: 5' ctg gaa tgg gtt gga tac att 3'
Olygonucleotide 2: 5' ggg gctagc tga gga gac ggt gac cgt ggt 3'
Olygonucleotide 3: 5' aat gta tcc aac cca ttc cag 3'
Olygonucleotide 4: 5' ggg gatatc cacc atg gaa agg cac tgg atc ttt ctc ttc ctg 3'

All these mutations were verified by sequence. The genetic construction obtained was denominated 14F7hTVH.

In the case of the heavy chain there are different amino acids between the VH14F7 and the most similar human one that were not performed because these substitutions involved amino acids of the Vernier zone or are key positions in the antigen recognition.

For the light chain mutations in positions 39, 40, 41, 42 and 58 were performed, substituting R, T, H, E, I, for K, P, G, Q, and V respectively. The mutations were introduced in the same way as in the heavy chain. Below the olygonucleotide sequences used are described.

Olygonucleotides for the mutations 39, 40, 41, and 42 of the light chain.
Olygonucleotide 1: 5' tat caa caa aaa cca ggt cag tct cca agg 3'
Olygonucleotide 2: 5' agc gtcgac tta cgt ttc agc tcc agc ttg gtc cc 3'
Olygonucleotide 3: 5' cct tgg aga ctg acc tgg ttt ttg ttg ata 3'
Olygonucleotide 4: 5' ggg gatatc cacc atg gt(at) t(tc)c (ta)ca cct cag (at)t(ac) ctt gga ctt 3'

After verifying by sequence the previous mutations a mutation in position 58 was introduced in the DNAs bearing them substituting the amino acid I for V. Below are shown the olygonucleotides 1 and 2, and 3 and 4, used in this mutation. The overlapping of the PCRs products was performed as described before.

Olygonucleotides for mutation 58 of the light chain:
Olygonucleotide 1: 5' att tct ggg gtc ccc tcc agg 3'
Olygonucleotide 2: 5' agc gtcgac tta cgt ttc agc tcc agc ttg gtc cc 3'
Olygonucleotide 3: 5' cct gga ggg gac ccc aga aat 3'
Olygonucleotide 4: 5' ggg gatatc cacc atg gt(at) t(tc)c (ta)ca cct cag (at)t(ac) ctt gga ctt 3'

Once the mutation was performed it was verified by sequence.

The resulting genetic construction was denominated 14F7hTVK.

The VK and VH humanized regions were cloned in the vectors PAG4622 and PAH4604, forming the constructions 14F7hTVH-PAH4604 and 14F7hTVK-PAG4622, respectively. NSO cells were electroporated with 10 µg of each vector that carried the humanized variable regions 14F7hTVH-PAH4604 and 14F7hTVK-PAG4622, previously linealized by digestion with *Pvu* I. The electroporation process and detection of the clones that express the 14F7hT humanized antibody was identical to the one described for the chimeric antibody.

### Example 4: Effect of the 14F7 Ab on the tumor growth decrease in the in vivo angiogenesis model

The melanoma B16 tumor cell line mixed with matrix gel as angiogenesis inductor was used as model for in vivo angiogenesis

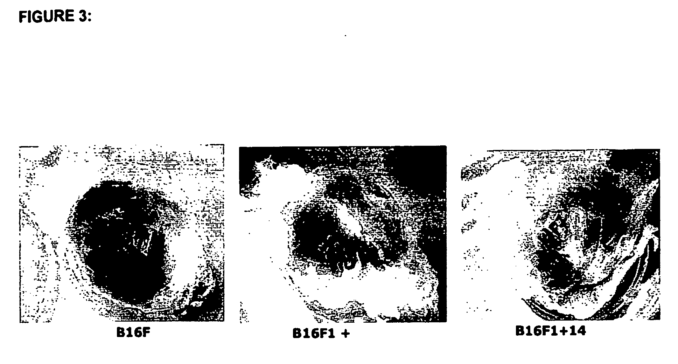

With the subcutaneous implantation of the tumor line (B16) with matrix gel in the middle ventral line of C57/BL6 male mice, the induction of the tumoral angiogenesis processes was measured. For this the melanoma B16 cell were mixed with 0.5 mL of matrix gel and 64 units/mL of heparin, alone, in the control group, or in combination with other antibodies, to be administered by subcutaneous injection in the abdominal region of the animals. Fifteen days after inoculation the animals were sacrificed and gel pearls were extracted along with the residues of dermis and epidermis and the size of the tumor is examined macroscopically. Figure 3 shows how the size of the tumor mass of the tumors of the mice treated with 14F7 decreased drastically.

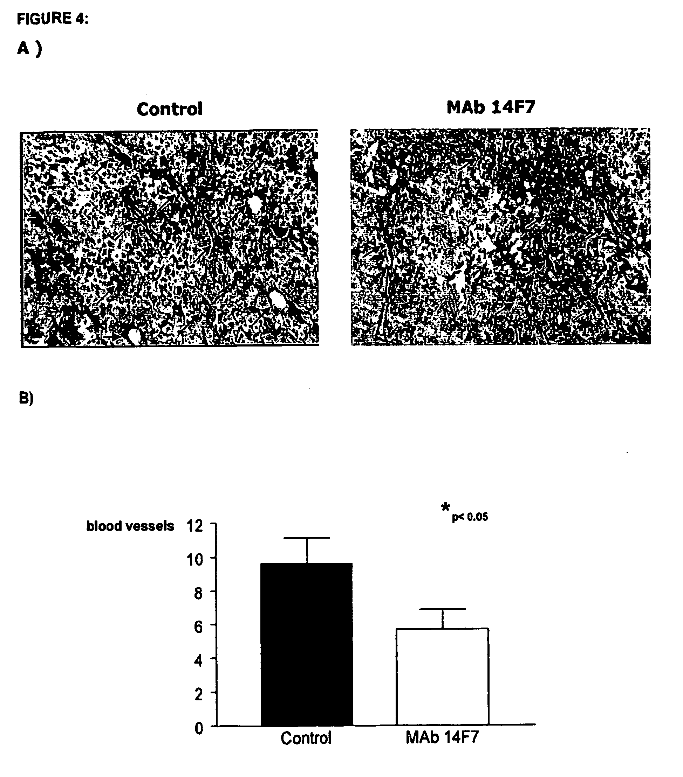

To distinguish the neovessels a histopathological analysis is performed of the gels stained with hetamtoxilin/eosin and with immunostaining with an anti PECAM Mab (endothelial marker). Figure 4, A and B, a decrease in the number of blood vessels is observed in the tissue sections of the animals treated with 14F7, when compared to the tumor sections of untreated animals.

### Example 5: Construction of antibody fragment libraries on filamentous phages from 14F7 hybridoma.

The messenger RNA was isolated with TriPure Isolation Reagent (Boehringer Mannheim, Germany) from 5.0 x 10⁶ 14F7 hybridoma cells and the complementary DNA was synthesized with Pro-STAR First Strand RT-PCR reagent kit (Stratagene, USA). The variable regions of the heavy and light chains derived from the hybridoma were amplified by 26 cycles of polymerase chain reaction in the following conditions: 50 seconds at 94º C, 1 minute at 55º C, 1 minute at 72º C. For this all the possible combinations of a kit of degenerated olygonucleotides were used (designed for hybridizing with a wide variaty of mice variable regions), which are shown in the following table:
Olygonucleotides used to amplify VH and VL. The degenerated positions appear in brackets in the sequence of the olygonucleotides. The sequences of the restriction sites are underlined.
5' VH *Apa* LI
5'... ttc tat tct cac agt gca cag (gc)ag gt(gt) cag ct(gt) ac(ag) cag tc(at) gga 3'
5'... ttc tat tct cac agt gca cag gc(ag) gt(ct) ca(ag) ctg cag cag ct(ct) ggg gc 3'
5'... ttc tat tct cac agt gca cag ga(ag) gtg aag ct(gt) (gc)t(gc) gag tct gg(ag) gga 3'
3' VH Sfi I
5'... ga acc agt act cca ggc ctg agg ggc cgc aga gac agt gac cag agt ccc 3'
5'... ga acc agt act cca ggc ctg agg ggc cga gga gac ggt gac tga ggt tcc 3'
5'... ga acc agt act cca ggc ctg agg ggc cga gga gac (gt)gt ga(gc) (ca) gt gga (gc)cc...3'
5' V_{L} Sal I
5'...gta ctc cag_tcg acg aca ttg tg(ca) tg(at) t(ac)c agt ctc c... 3'
5'... gta ctc cag tcg acg ata tcc aga tga c(ac)c a(ga)a ct(ac) c... 3'
5'... gta ctc cag tcg ac(cg) aaa ttg t(tg)c tca ccc agt ctc c... 3'
3' V_{L} Not I
5'...aag gaa aaa agc ggc cgc ttt (tc)a(tg) (tc)tc cag ctt ggt... 3'
5'...aag gaa aaa agc ggc cgc ttt (tg)a(tg) ctc caa ctt gt(gt)... 3'

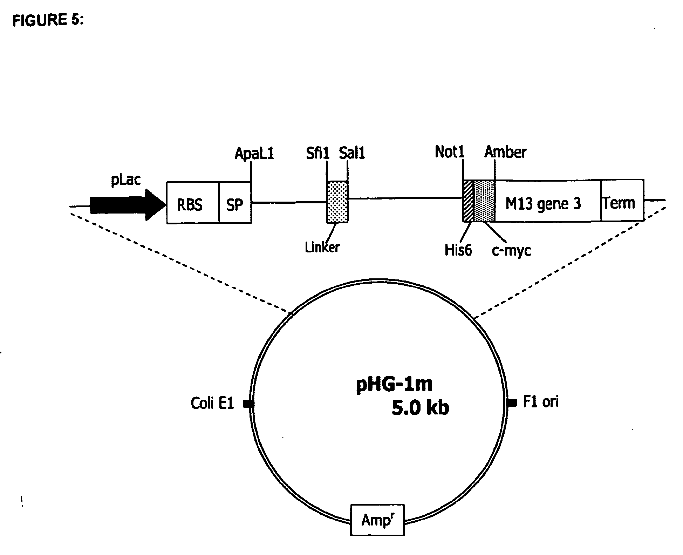

The variable regions of the amplified heavy chains were purified and sequentially digested with the restriction enzymes Sfi I and ApaLI. The digestion products were purified and linked with the DNA of the phagomidium vector pHG-1m (Heber Biotec S.A., Cuba) (Figure 5). With the resulting genetic constructions bacterias of the TG1 of *E.coli* strain were transformed by electroporation. The group of transformed bacteria conformed a semi-library of heavy chains variable regions derived from the 14F7 hybridoma with a moderate size of 2.3 x 10⁴ members. The plasmid DNA of the semi-library was purified and was sequentially digested with Not I and Sal I enzymes. This DNA was linked separetly with four collections of light chain variable regions previously digested with the same enzymes. Bacterias of the TG1 strain were transformed with these new genetic constructions and four independent libraries were obtained. One of them was a mini-library of limited diversity containing the varialbe regions both of the heavy and light chains derived from the 14F7 hybridoma with a size of 1.6 x 10⁶ individuals. The other three were chain exchange libraries that incorporated diverse collections of variable regions of light chains obtained previously from mice (kappa isotype) and humans (kappa and lambda isotypes) and their size were of 1.0 x 10⁷, 1.4 x 10⁶, and 1.2 x 10⁶ members respectively.

### Example 6: Isolation of clones producing antibody fragments against the N-glycolil GM3.

Phages carrying the antibody fragments from 92 clones taken at random of each library were produced, in microtitulation plate wells according to procedures described before (Marks, J. D. et. al, J. Mol. Biol. 222,581-597. 1991). Its specificity was evaluated in an ELISA on polyvinyl chloride plates (Costar, USA) coated with N-glycolil GM3 and N-acetyl GM3 at 1µg/ml. The bound phages were detected with an anti-M13 monoclonal antibody conjugated with peroxidase (Amersham, Sweden). In addition to the characterization of individual clones picked at random, the selection of the phages with capacity of binding to the N-glycolil GM3 from a mixture of phages produced by a group of bacterias that conform each library was performed. For this, phages were produced using the auxiliary phage M13 K07, they were purified by precipitation with polyethylenglicol according to the procedure referred and they were put in contact with the N-glycolil GM3 bound to a plastic surface (Inmuno tubes, Nunc, Denmark). The tubes were washed extensively and the phages eluted in presence of triethylamine 100 mmol/l. The elutate was neutralized and used to infect bacterias of the TG1 of *E.coli* strain. From the group of infected bacterias phages carrying the antibody were produced and purified again that were used as starting material for a new selection round in identical conditions to those described. After four selection cycles 92 colonies of bacteria infected with the phages resulting from each library were taken at random and the recognition of the N-glycolil GM3 was performed in a similar form as described for the evaluation of the clones taken at random from the libraries.

From the direct analysis of the clones of the libraries producing clones were isolated from phages carrying the antibody fragments that recognize the N-glycolil GM3, that were found in a variable frequency in the three chain exchange libraries. No positive clone was found in the mini-library formed exclusively by variable regions derived from the 14F7 hybridoma. After the selection cycles additional clones were isolated from the chain exchange libraries and none were found in the mini-library of the original sequences. The following table shows the quantity of clones producing antibody fragments that recognize the N-glycolil GM3 and the frequency that it represents of the total number of clones analyzed from each library. Only one clone produced phages that presented cross reactivity with the N-acetyl GM3.

| Library | Frequency of positive clones (direct screening) | Frequency of positive clones (four selection cycles) |
|---|---|---|
| Mini-library hybridoma 14F7 | 0/92 | 0/92 |
| Exchange of light kappa murine chains | 31/92 (33.70%) * | 52/92 (56.52%) |
| Exchange of light kappa human chains | 2/92 (2.17%) | 8/92 (17.39%) |
| Exchange of light lambda human chains | 4/92 (4.35%) | 12/92 (13.04%) |

One clone with cross reactivity with the N-acetyl GM3.

### Example 7. Characterization of the antibody fragments derived from the 14F7 that recognize the N-glycolil GM3

The variable regions of 11 antibody fragments were completely sequenced with an automatic sequencer ALF Express II (Pharmacia, Sweden), using olygonucleotides that hybridize in the regions of the pHG-1m vector that flank the extremes 5' and 3' of the sequences that codify for the inserted antibody fragments. It was confirmed that the sequence of the variable region of all the heavy chains corresponds to the one reported for the 14F7, changes were only presented in the framework 1 and framework 4 regions, presumable introduced by the use of degenerated olygonucleotides in the PCR. The reset of the sequences included the three CDRs had no variations. On the contrary, the sequences of the variable regions of the light chains of the 11 clones were all different from the one of the 14F7 and were grouped in 5 groups of different sequences, two of murine origin and one of human origin (kappa isotype) and two other of human origin but of lambda isotype. One clone representative of each sequence group was taken for its latter characterization.

The following table shows the differences between the variable regions of the light chains of the selected clones with respect to their original identity, their origin and isotype and the the classification in subgroups according to the Kabat classification.

| | VL Origin | Isotype | Identity with V_{L} 14F7 | Subgroup |
|---|---|---|---|---|
| AcM 14F7 | Murine | kappa | - | V |
| ScFv 2Am | Murine | kappa | 59.46% | III |
| ScFv 3Fm | Murine | kappa | 59.81% | V |
| ScFv 7Bhk | Human | kappa | 60.74% | I |
| ScFv 7Ahl | Human | lambda | 35.45% | I |
| ScFv 8Bhl | Human | lambda | 37.04% | III |

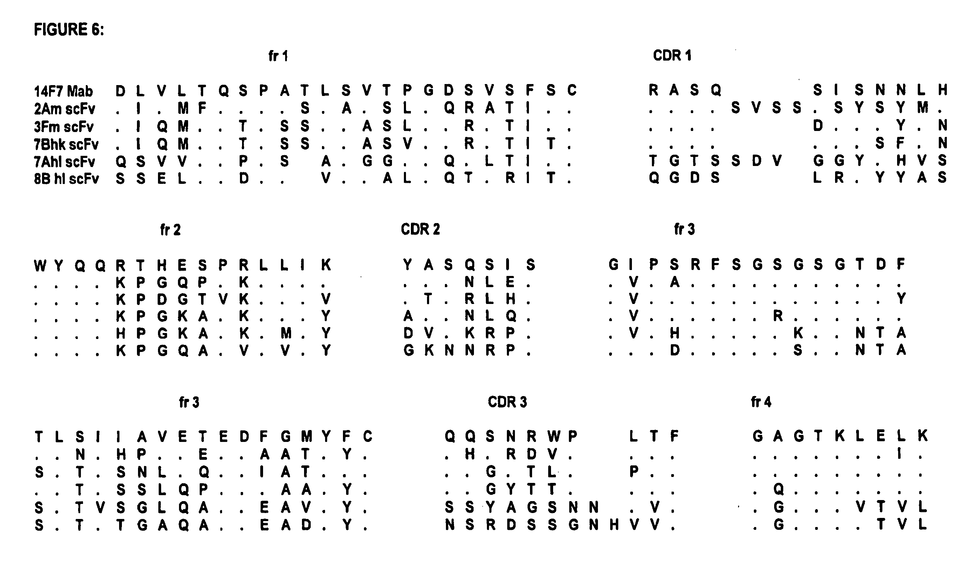

The differences between the variable regions of the light chains of the clones and the ones reported originally for the 14F7 antibody are localized along all the sequence including the three complementary determinant regions and are shown below (Figure 6).
**Fr1** 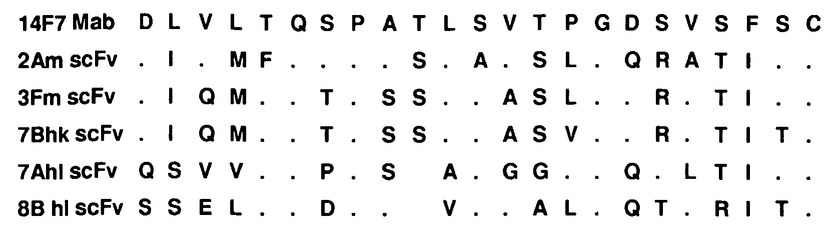 **CDR1** 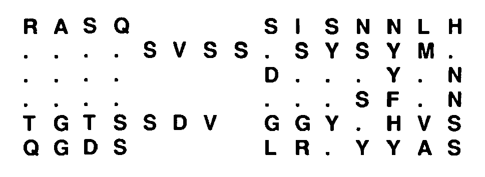 **Fr 2** 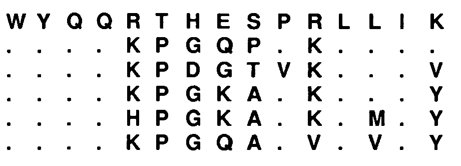 **CDR 2** 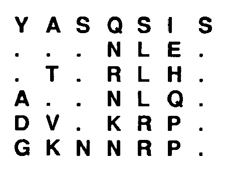 **Fr 3** 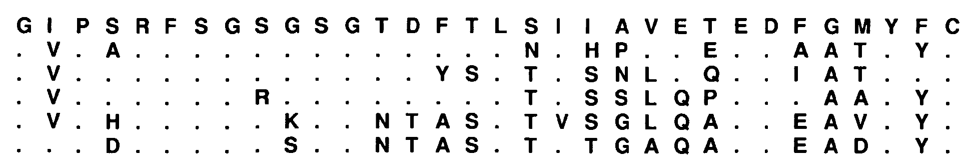 **CDR 3** 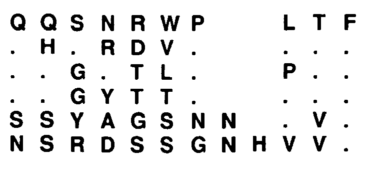 **Fr 4**

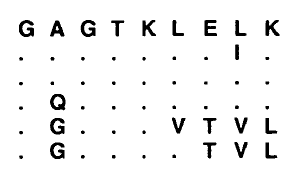

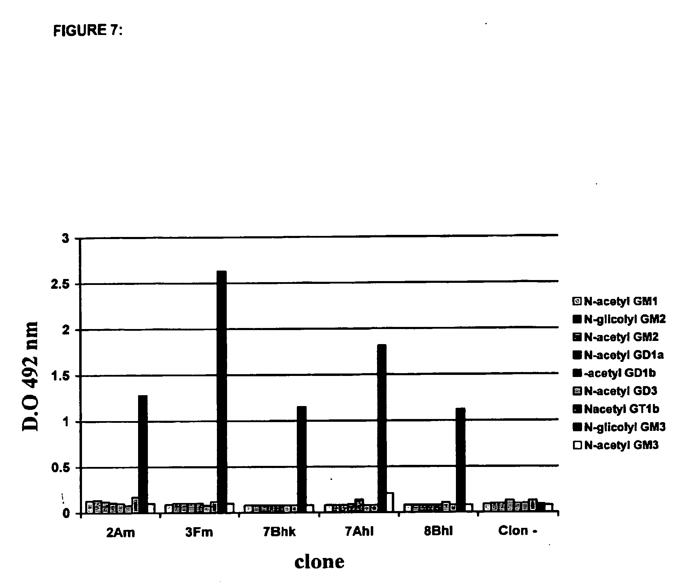

The specificity of the binding confirmed by ELISA according to the method described by using as coating in addition to the N-glycolil GM3 and the N-acetyl GM3, a panel of related gangliosides that included various N-acetylated gangliosides and one N-glycolilated ganglioside. Figure 7 shows the recognition of the different antigens by the 5 clones producing characterized antibody fragments. Only the binding to the target antigen N-glycolil GM3 was detected.

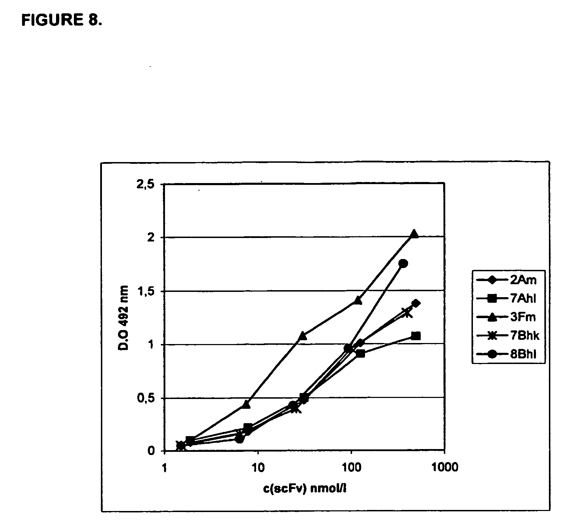

The production of the soluble antibody fragments (outside the context of the phages) by the clones was induced in presence of isopropil-thiogalactopiranoside at 1mmol/l. The supernatant were collected and the periplasmatic fractions were obtained according to the established procedures (de Haard, H. J.Biol. Chem. 274, 18218-18230, 1999). Recognition activity of the N-glycolil GM3 was detected both in the culture supernatants as in the periplasmatic fractions through an ELISA on plates coated with said antigen similar to the one described but with the following modification. The bound antibody fragments were detected using the 1E10 monoclonal antibody (directed against the c-*myc* peptide fused in the genetic construction to the antibody fragments) at 10 µg/ml and an anti mice immunoglobulins conjugate (Sigma, USA). The antibody fragments were purified from the periplasmatic fractions in a single affinity chromatography to metallic ions using the matrix HIS-Select HC Nickel Affinity Gel (Sigma, USA) according to the manufacturer's instructions. Figure 8 shows the activity determined by ELISA of the purified soluble fragments.

### Brief description of the Figures.

**Figure 1****:** Shows the amino acidic sequence of the variable regions VH (a) and VK (b) of the 14F7 Mab, and the respective most homologous human sequence. The CDRs are underlined and the amphypatic regions and potential T epitopes are shadowed. The sequence resulting of the humanization of the variable regions with the mutations performed is also shown.
**Figure 2****:** Recognition of the 14F7Q antibody measured by competitive ELISA.
   Axis X: Expresses the antibodies concentrations.
   Axis Y: Absorbance measured at 492 nm

   The ior C5 monoclonal antibody was used as negative control.
**Figure 3****:** Effect of the 14F7 monoclonal antibody on the growth of murine tumors (melanoma B16 tumor cell line).
**Figure 4****:** A) Immunohistochemical study of the effect of the 14F7 monoclonal antibody on the formation of blood vessels. The arrows indicate the immunostained vessels. B) Inhibition of angiogenesis induced by the 14F7 monoclonal antibody.
**Figure 5****:** Diagram of the pHG-1 m vector.
**Figure 6****:** Protein sequence of the VL regions of the 14F7 monoclonal antibody and the selected Fv single chain fragments. The points indicate homology with the sequence of the VL of the 14F7.
**Figure 7****:** Recognition specificity by the phages carrying the antibody fragments. The inhibition of the binding of the 14F7 Ab to the ganglioside by the exposed fragments in the phages is observed. The plates are coated with N-glycolil GM3 and the phages were incubated with viral particles and varias concentrations of 14F7.
**Figure 8****.** Recognition of the N-glycolil GM3 by the soluble purified fragments.

## Claims

1. A recombinant humanized antibody that recognizes the NGcGM3 ganglioside comprising the constant region of the IgG1human heavy chain and the constant region of the Ck human light chain and heavy and light chain variable regions from the murine 14F7 monoclonal antibody produced by the hybridoma with the deposit ECACC 98101901, wherein the murine 14F7 monoclonal antibody comprises the sequences of the hyper variable regions (CDRs) of the heavy and light chains shown below:
HEAVY CHAIN
CDR1: SYWIH
CDR2: YIDPATAYTESNQKFKD
CDR3: ESPRLRRGIYYYAMDY
LIGHT CHAIN
CDR1: RASQSISNNLH
CDR2: YASQSIS
CDR3: QQSNRWPLT;
and the murine 14F7 monoclonal antibody comprises the sequences of the framework regions (FRs) of the heavy and light chains shown below:
HEAVY CHAIN
FR1: QVQLQQSGNELAKPGASMKMSCRASGYSFT
FR2: WLKQRPDQGLEWIG
FR3: KAILTADRSSNTAFMYLNSLTSEDSAVYYCAR
FR4: WGQGTTVTVSS
LIGHT CHAIN
FR1: DLVLTQSPATLSVTPGDSVSFSC
FR2: WYQQRTHESPRLLIK
FR3: GIPSRFSGSGSGTDFTLSIISVETEDFGMYFC
FR4: FGAGTKLELKRA;
wherein the recombinant humanized antibody is **characterized by** containing the sequence of the variable region of the heavy chain of the murine 14F7 monoclonal antibody and a light chain variable region whose sequence is the following:
Fr1
D I Q M T Q T P S S L S A S L G D R V T I S C
CDR1
R A S Q D I S N Y L N
Fr 2
W Y Q Q K P D G T V K L L I V
CDR 2
Y T S R L H S
Fr 3
G V P S R F S G S G S G T D Y S L T I S N L E Q E D I A T Y F C
CDR 3
Q Q G N T L P P T F
Fr 4
G A G T K L E L K
or:
Fr1
D I Q M T Q T P S S L S A S V G D R V T I T C
CDR1
R A S Q S I S S F L N
Fr 2
W Y Q Q K P G K A P K L L I Y
CDR 2
A A S N L Q S
Fr 3
G V P S R F S G R G S G T D F T L T I S S L Q P E D F A A Y Y C
CDR 3
Q Q G Y T T P L T F
Fr 4
G Q G T K L E L K
or:
Fr1
Q S V V T Q P P S A S G G P G Q S L T I S C
CDR 1
T G T S S D V G G Y N H V S
Fr 2
W Y Q Q H P G K A P K L MI Y
CDR 2
D V S K R P S
Fr 3
G V P H R F S G S K S G N T A S L T V S G L Q A E D E A V Y Y C
CDR 3
S S Y A G S N N L V F
Fr 4
GGGTKVTVL

2. Single chain Fv fragment that recognizes the NGcGM3 ganglioside derived from the murine 14F7 monoclonal antibody produced by the hybridoma with deposit number ECACC 98101901 **characterized by** containing the sequence of the variable region of the heavy chain of the 14F7 monoclonal antibody and a variable region of the light chain of a murine antibody, whose sequence is as follows:
Fr1: DIQMTQTPSSLSASLGDRVTISC
CDR 1: RASQDISNYLN
Fr2: WYQQKPDGTVKLLIV
CDR2: YTSRLHS
Fr3: GVPSRFSGSGSGTDYSLTISNLEQEDIATYFC
CDR3: QQGNTLPPTF
Fr4: GAGTKLELK,
or
Fr1: DIQMTQTPSSLSASVGDRVTITC
CDR1: RASQSISSFLN
Fr2: WYQQKPGKAPKLLIY
CDR2: AASNLQS
Fr3: GVPSRFSGRGSGTDFTLTISSLQPEDFAAYYC
CDR3: QQGYTTPLTF
Fr4: GQGTKLELK,
or
Fr1: QSVVTQPPSASGGPGQSLTISC
CDR1: TGTSSDVGGYNHVS
Fr2: WYQQHPGKAPKLMIY
CDR2: DVSKRPS
Fr3: GVPHRFSGSKSGNTASLTVSGLQAEDEAVYYC
CDR3: SSYAGSNNLVF
Fr4: GGGTKVTVL.

3. A cell line expressing the recombinant antibody or the single chain Fv fragment of claims 1 or 2.

4. Pharmaceutical composition for use in the treatment of malignant tumors comprising the recombinant antibody or the single chain Fv fragment of claims 1 or 2, and an appropriate excipient.

5. Pharmaceutical composition of claim 4 for use in the treatment of malignant breast tumors and melanomas and their metastases and recurrences.

## Patentansprüche

1. Rekombinanter, humanisierter Antikörper, der das NGcGM3-Gangliosid erkennt, umfassend die konstante Region der humanen IgG1-schweren Kette und die konstante Region der humanen Ck-leichten Kette und variable Regionen der schweren und leichten Kette des murinen, monoklonalen 14F7-Antikörpers, produziert von dem Hybridom mit der Hinterlegung ECACC 98101901, wobei der murine, monoklonale 14F7-Antikörper die Sequenzen der hypervariablen Regionen (CDRs) der unten aufgeführten schweren und leichten Ketten umfasst:
SCHWERE KETTE
CDR1: SYWIH
CDR2: YIDPATAYTESNQKFKD
CDR3: ESPRLRRGIYYYAMDY
LEICHTE KETTE
CDR1: RASQSISNNLH
CDR2: YASQSIS
CDR3: QQSNRWPLT;
und der murine, monoklonale 14F7-Antikörper die Sequenzen der Rahmenregionen (FRs) der unten aufgeführten schweren und leichten Ketten umfasst:
SCHWERE KETTE
FR1: QVQLQQSGNELAKPGASMKMSCRASGYSFT
FR2: WLKQRPDQGLEWIG
FR3: KAILTADRSSNTAFMYLNSLTSEDSAVYYCAR
FR4: WGQGTTVTVSS
LEICHTE KETTE
FR1:DLVLTQSPATLSVTPGDSVSFSC
FR2: WYQQRTHESPRLLIK
FR3: GIPSRFSGSGSGTDFTLSIISVETEDFGMYFC FR4:FGAGTKLELKRA;
wobei der rekombinante humanisierte Antikörper **dadurch gekennzeichnet ist, dass** er die Sequenz der variablen Region der schweren Kette des murinen, monoklonalen 14F7-Antikörpers und einer variablen Region der leichten Kette mit folgender Sequenz beinhaltet:
Fr1
D I Q M T Q T P S S L S A S L G D R V T I S C
CDR1
R A S Q D I S N Y L N
Fr2
W Y Q Q K P D G T V K L L I V
CDR2
Y T S R L H S
Fr3
G V P S R F S G S G S G T D Y S L T I S N L E Q E D I A T Y F C
CDR3
Q Q G N T L P P T F
Fr4
G A G T K L E L K
oder:
Fr1
D I Q M T Q T P S S L S A S V G D R V T I T C
CDR1
R A S Q S I S S F L N
Fr2
W Y Q Q K P G K A P K L L I Y
CDR2
A A S N L Q S
Fr3
G V P S R F S G R G S G T D F T L T I S S L Q P E D F A A Y Y C
CDR3
Q Q G Y T T P L T F
Fr4
G Q G T K L E L K
oder:
Fr1
Q S V V T Q P P S A S G G P G Q S L T I S C
CDR1
TGTSSDVGGYNHVS
Fr2
W Y Q Q H P G K A P K L M I Y
CDR2
DVSKRPS
Fr3
G V P H R F S G S K S G N T A S L T V S G L Q A E D E A V Y Y C
CDR3
S S Y A G S N N L V F
Fr4
GGGTKVTVL

2. Fv-Einzelkettenfragment, welches das von dem murinen, monoklonalen 14F7-Ganglosid-Antikörper, produziert von dem Hybridom mit Hinterlegungsnummer ECACC 98101901, abgeleitete NGcGM3-Gangliosid erkennt, **dadurch gekennzeichnet, dass** es die Sequenz der variablen Region der schweren Kette des monoklonalen 14F7-Antikörpers und eine variable Region der leichten Kette eines murinen Antikörpers mit folgender Sequenz beinhaltet:
Fr1: DIQMTQTPSSLSASLGDRVTISC
CDR1: RASQDISNYLN
Fr2: WYQQKPDGTVKLLIV
CDR2: YTSRLHS
Fr3: GVPSRFSGSGSGTDYSLTISNLEQEDIATYFC
CDR3: QQGNTLPPTF
Fr4: GAGTKLELK,
oder
Fr1: DIQMTQTPSSLSASVGDRVTITC
CDR1: RASQSISSFLN
Fr2: WYQQKPGKAPKLLIY
CDR2: AASNLQS
Fr3: GVPSRFSGRGSGTDFTLTISSLQPEDFAAYYC
CDR3: QQGYTTPLTF
Fr4: GQGTKLELK,
oder
Fr1: QSVVTQPPSASGGPGQSLTISC
CDR1: TGTSSDVGGYNHVS
Fr2: WYQQHPGKAPKLMIY
CDR2: DVSKRPS
Fr3: GVPHRFSGSKSGNTASLTVSGLQAEDEAVYYC
CDR3: SSYAGSNNLVF
Fr4: GGGTKVTVL.

3. Zelllinie, die den rekombinanten Antikörper oder das Fv-Einzelkettenfragment der Ansprüche 1 oder 2 exprimiert.

4. Pharmazeutische Zusammensetzung zur Verwendung bei der Behandlung maligner Tumore, umfassend den rekombinanten Antikörper oder das Fv-Einzelkettenfragment der Ansprüche 1 oder 2 und einen geeigneten Hilfsstoff umfasst.

5. Pharmazeutische Zusammensetzung nach Anspruch 4 zur Verwendung bei der Behandlung maligner Brusttumore und Melanome sowie deren Metastasen und Wiederkehr.

## Revendications

1. Anticorps humanisé recombinant qui reconnaît le ganglioside NGcGM3 comprenant la région constante de la chaîne lourde de IgG1 humaine et la région constante de la chaîne légère de Ck humaine et des régions variables de chaînes lourdes et légères provenant de l'anticorps monoclonal 14F7 murin produit par l'hybridome ayant le dépôt ECACC 98101901, dans lequel l'anticorps monoclonal 14F7 murin comprend les séquences des régions hyper variables (CDR) des chaînes lourdes et légères représentées ci-dessous :
CHAINE LOURDE :
CDR1 : SYWIH
CDR2 : YIDPATAYTESNQKFKD
CDR3 : ESPRLRRGIYYYAMDY
CHAINE LEGERE :
CDR1 : RASQSISNNLH
CDR2 : YASQSIS
CDR3 : QQSNRWPLT
et l'anticorps monoclonal 14F7 murin comprend les séquences des régions d'ossature (FR) des chaînes lourdes et légères représentées ci-dessous :
CHAINE LOURDE :
FR1 : QVQLQQSGNELAKPGASMKMSCRASGYSFT
FR2 : WLKQRPDQGLEWIG
FR3 : KAILTADRSSNTAFMYLNSLTSEDSAVYYCAR
FR4 : WGQGTTVTVSS
CHAINE LEGERE :
FR1: DLVLTQSPATLSVTPGDSVSFSC
FR2: WYQQRTHESPRLLIK
FR3: GIPSRFSGSGSGTDFTLSSIISVETEDFGMYFC
FR4: FGAGTKLELKRA
dans lequel l'anticorps humanisé recombinant est **caractérisé en ce qu'**il contient la séquence de la région variable de la chaîne lourde de l'anticorps monoclonal 14F7 murin et d'une région variable de chaîne légère dont la séquence est la suivante :
Fr1
DIQMTQTPSSLSASLGDRVTISC
CDR1
R A S Q D I S N Y L N
Fr2
W Y Q Q K P D G T V K L L I V
CDR2
YTSRLHS
Fr3
GV P S R F S G S G S GT D Y S L T I S N L E Q E D I A T Y F C
CDR3
Q Q G N T L P P T F
Fr4
GAGTKLELK
ou:
Fr1
D I Q M T Q T P S S L S A S V G D R V T I T C
CDR1
R A S Q S I S S F L N
Fr 2
W Y Q Q K P G K A P K L L I Y
CDR2
A A S N L Q S
Fr 3
G V P S R F S G R G S G T D F T L T I S S L Q P E D F A A Y Y C
CDR3
Q Q G Y T T P L T F
Fr 4
G Q G T K L E L K
ou:
Fr1
Q S V V T Q P P S A S G G P G Q S L T I S C
CDR1
TGTSSDVGGYNHVS
Fr 2
W Y Q Q H P G K A P K L M I Y
CDR2
DVSKRPS
Fr 3
G V P H R F S G S K S G N T A S L T V S G L Q A E D E A V Y Y C
CDR3
SSYAGSNNLVF
Fr 4
GGGTKVTVL

2. Fragment Fv à simple chaîne qui reconnaît le ganglioside NGcGM3 dérivé de l'anticorps monoclonal 14F7 murin produit par l'hybridome ayant le numéro de dépôt ECACC 98101901 **caractérisé en ce qu'**il contient la séquence de la région variable de la chaîne lourde de l'anticorps monoclonal 14F7 et une région variable de la chaîne légère d'un anticorps murin, dont la séquence est comme suit :
Fr1 : D I Q M T Q T P S S L S A S L G D R V T I S C
CDR 1: R A S Q D I S N Y L N
Fr2 : W Y Q Q K P D G T V K L L I V
CDR2 : Y T S R L H S
Fr3 : G V P S R F S G S G S G T D Y S L T I S N L E Q E D I A T Y F C
CDR3 :QQGNTLPPTF
Fr4 : G A G T K L E L K,
ou
Fr1 : D I Q M T Q T P S S L S A S V G D R V T I T C
CDR1 : R A S Q S I S S F L N
Fr2: WYQQKPGKAPKLLIY
CDR2 : A A S N L Q S
Fr3 : G V P S R F S G R G S G T D F T L T I S S L Q P E D F A A Y Y C
CDR3 :QQGYTTPLTF
Fr4 : G Q G T K L E L K,
ou
Fr1 : Q S V V T Q P P S A S G G P G Q S L T I S C
CDR1 :TGTSSDVGGYNHVS
Fr2: WYQQHPGKAPELMIY
CDR2 : D V S K R P S
Fr3 : G V P H R F S G S K S G N T A S L T V S G L Q A E D E A V Y Y C
CDR3:SSYAGSNNLVF
Fr4 : G G G T K V T V L.

3. Ligne cellulaire exprimant l'anticorps recombinant ou le fragment Fv à simple chaîne des revendications 1 ou 2.

4. Composition pharmaceutique destinée à être utilisée dans le traitement de tumeurs malignes comprenant l'anticorps recombinant ou le fragment Fv à simple chaîne des revendications 1 ou 2, et un excipient approprié.

5. Composition pharmaceutique selon la revendication 4 destinée à être utilisée dans le traitement de tumeurs malignes du sein et de mélanomes et de leurs métastases et récurrences.
